# EUROPEAN PATENT APPLICATION

(11) **EP 4 461 217 A1**
(43) Date of publication of application: **13.11.2024**
(21) Application number: 24174479.6
(22) Date of filing: 07.05.2024
(51) Int. Cl.: A61B 5/145, A61B 5/20, A61B 5/00, A61B 5/1455, A61B 5/1459

(54) **MONITORING A KIDNEY-RELATED CONDITION**

(30) Priority: 08.05.2023 US 202318313713
(71) Applicant: Indigo Diabetes N.V., 9052 Gent (BE)
(72) Inventor: ORDONEZ ORELLANA, Juan Sebastian, 9000 Gent (BE); DELBEKE, Danaë, 9050 Gentbrugge (BE); SEGERS, Tom, 9052 Gent (BE)
(74) Representative: Winger

(57) **Abstract**

A monitoring system 100 for monitoring a kidney-related condition of a living creature. The monitoring system 100 comprises an implantable sensor 110 for repetitively measuring creatinine information in bodily tissue or bodily fluids of the living creature and for storing said creatinine information, and a processor 120 programmed for determining, from the stored information, a kidney-related parameter for the living creature.

## Description

### Field of the invention

The invention relates to the field of health monitoring. More particularly, the present invention relates to systems and methods for monitoring kidney related parameters for monitoring kidney related diseases, kidney transplants, etc., using an implantable device for continuously, time-dependent, stepwise, detection of one or more related metabolism analytes.

### Background of the invention

Diabetic Kidney Disease (DKD), also known as diabetic nephropathy, is a complication of diabetes in which high blood sugar levels damage the kidneys' filtering system. Over time, this damage can progress and lead to chronic kidney disease (CKD) and kidney failure.

In people with diabetes, high levels of blood sugar can damage the tiny blood vessels in the kidneys that filter waste products. This damage can cause the kidneys to become less efficient at filtering waste, which can lead to a build up of waste products in the blood and damage to other organs in the body.

Symptoms of DKD may not be noticeable in the early stages, but as the disease progresses, patients may experience symptoms such as swelling in the legs and feet, fatigue, and difficulty sleeping. The progression of DKD can be slowed or halted by controlling blood sugar levels, blood pressure, and cholesterol levels, and by avoiding smoking and maintaining a healthy diet and exercise routine.

Early detection and management of DKD are essential to prevent the progression of the disease and reduce the risk of complications such as kidney failure and cardiovascular disease. People with diabetes should have regular check-ups with their healthcare provider to monitor kidney function and screen for signs of DKD.

Early detection of DKD involves a combination of screening tests and monitoring of kidney function over time. The following are some common methods for detecting DKD:
Urine albumin test: A urine albumin test measures the level of albumin in the urine. High levels of albumin in the urine may indicate early-stage kidney damage
Blood creatinine test: A blood creatinine test measures the level of creatinine in the blood, which is a waste product that is filtered by the kidneys. High levels of creatinine in the blood may indicate decreased kidney function
Estimated glomerular filtration rate (eGFR): The eGFR is calculated based on the results of a blood creatinine test and other factors such as age, sex, and race. The eGFR provides an estimate of how well the kidneys are functioning
Kidney biopsy: A kidney biopsy may be performed if there is suspicion of advanced DKD or if the results of other tests are inconclusive.

It is recommended that people with diabetes have regular check-ups with their healthcare provider to monitor kidney function and screen for signs of DKD. The frequency of testing may vary depending on the stage of DKD and other factors such as age, sex, and overall health. Early detection and management of DKD are essential to prevent the progression of the disease and reduce the risk of complications such as kidney failure and cardiovascular disease. Blood tests and urine tests are the most used diagnostic tests, followed by imaging tests and biopsy.

More generally, chronic kidney disease can be split in different segments such as for example diabetes nephropathy, glomerular diseases, hypertensive nephropathy, polycystic kidney disease and others. Furthermore, monitoring of kidney related parameters can also be used for following up kidney transplants or for following the effects of dialysis sessions.

There is still a need for good monitoring systems and methods of kidney related information.

### Summary of the invention

It is an object of the present invention to provide methods and systems allowing accurate monitoring of kidney related parameters.

It is an advantage of embodiments of the present invention that by accurate monitoring, initiation of dialysis can be delayed. The cost of dialysis can thus be reduced. The cost can vary depending on a number of factors, including the patient's age, overall health status, and the severity of their kidney disease.

While monitoring creatinine levels in patients with CKD is an essential aspect of care, it may not directly result in cost savings in the short term. However, regular monitoring of creatinine levels and other indicators of kidney function can help to detect changes in kidney function over time and identify patients who are at risk for disease progression. Early detection and management of CKD can help to slow the progression of the disease, which may lead to cost savings over the long term.

For example, if a patient's CKD is detected early and managed appropriately, it may be possible to delay or prevent the need for more costly interventions such as dialysis or kidney transplantation. Additionally, managing CKD can help to reduce the risk of complications such as cardiovascular disease, which can be expensive to treat.

It is important to note that delaying the initiation of dialysis is not always appropriate or feasible for all patients with kidney disease. The decision to initiate dialysis is typically based on a variety of factors, including the patient's overall health status, the severity of their kidney disease, and their individual treatment goals and preferences.

It is an advantage according to embodiments of the present invention that such health monitoring can be performed in a real individualized and personal manner. It is an advantage of embodiments of the present invention that health monitoring can be based on a metabolic fingerprint, providing information regarding metabolism analytes in the human body, in the present application being providing at least creatinine information for deriving a kidney related parameter thereof.

It is an advantage of embodiments of the present invention that a personal health monitoring system is provided allowing to have "the doctor's visit in your pocket". It thereby is an advantage that the special conditions of a doctor's visit and the affected results thereby are exchanged for a real continuous monitor that measures in real environmental situations, under real conditions of the activation of the metabolism and of the autonomous nervous system controls.

It is also an object of the present invention to provide a platform for assisting in monitoring health of living creatures and/or in assisting in assessment of the metabolic impact of a selected lifestyle, drug use, ..... on kidney related parameters.

The object and optionally also advantages are obtained by methods and systems according to aspects of the present invention.

The present invention relates to a monitoring system for monitoring a kidney-related condition of a living creature, the health monitoring system comprising
an implantable sensor for repetitively measuring creatinine information in bodily tissue or bodily fluids of the living creature and for storing said creatinine information,
a processor programmed for determining, from the stored information, a kidney-related parameter for the living creature.

In some embodiments, a kidney-related parameter may be related to a pre-operative or postoperative kidney transplant status, end stage renal disease, a status or degree of dialysis, a stage of lupus nephritis, a status or degree of chronic kidney disease, etc.

The kidney may be one of an artificial kidney, a transplanted kidney or an own kidney of the living creature.

The implantable sensor may be configured for substantially simultaneously measuring glucose related information in said bodily tissue or bodily fluids and for storing said glucose related information. The processor may be programmed for determining a kidney-related parameter from the combination of the stored creatinine information and glucose related information.

It is an advantage of at least some embodiments that effects of diabetes on kidney function and vice versa can be monitored and/or evaluated.

It is an advantage of embodiments of the present invention that methods and systems are provided allowing an accurate evaluation of a plurality of metabolism analytes in a living creature by monitoring thereof and by providing data being captured at the same moment in time, i.e. by providing paired data.

The implantable sensor may be configured for obtaining spectrophotometric sensing data and for deriving the creatinine information and the glucose (e.g. blood sugar) related information from the same spectrophotometric sensing data.

It is an advantage of at least some embodiments that information regarding creatinine and glucose can be captured from the same sensing data, optimally allowing to evaluate correlation between the obtained information.

The monitoring system furthermore may comprise an output port for providing an output to a user, a medical practitioner or a medical team. The monitoring system may be programmed for providing personalised recommendations regarding the kidney-related parameter to the user through the output port.

The creatinine information may be a creatinine concentration and the processor may be configured for indicating whether the obtained concentration information is within a predetermined range, for providing the user with a warning for consulting a medical practitioner or for providing a warning to a medical practitioner of the user.

The processor may be configured for comparing the creatinine information with a predetermined range, whereby the predetermined range is based on previously measured creatinine information from the living creature.

The processor may be adapted for determining an estimated glomerular filtration rate (eGFR).

The living creature may be a human being and the system may comprise an input port configured for receiving information regarding at least one of an age, length, weight, gender and/or ethnical characteristic of the human being. The processor may be configured for taking into account one, more or all of said at least one of age, length, weight, gender and/or ethnical characteristic of the human being for determining the estimated glomerular filtration rate.

The monitoring system may be adapted for indicating a degree or stage of diabetes nephropaty and/or for indicating that a consult with a medical practitioner is advised.

The monitoring system may be adapted for indicating, after a kidney transplant in a patient, a degree of acceptance of a transplanted kidney by the body of the patient.

The system may be configured for time-dependent monitoring during a dialysis session and the processor may be configured for indicating when a blood condition is sufficient to stop the dialysis session.

The system may be configured to measure the creatinine related information and/or optional one or more other metabolism analytes at different moments in time or continuously. The term 'continuous' or 'continuously' in relation to the invention should be construed as meaning `regularly without requiring regular user intervention', the sampling rate can be a fixed number of measurements per time frame or varied by an integrated controller. In embodiments according to the invention, the implantable sensor may comprise an integrated controller which is provided for controlling the sensing means at a variable sampling rate. In embodiments, the integrated controller may be provided for detecting a variability level in said sensor data and adapting said variable sampling rate according to said detected variability level, for example by reducing the sample rate if a low variability level (beneath a certain threshold) is detected. In other embodiments, the sampling rate may also be controlled by the monitoring device. By reducing the sampling rate, for example when it is expected that the sensor data will not vary much over a longer period of time, energy consumption of the sensing means of the implanted sensor can be reduced and battery life can possibly be extended.

The system may be configured to, based on said determining for creatinine related information or for other metabolism analytes whether the obtained concentration information is within a predetermined range, trigger additional measurements of the creatinine related information or one or more optional additional metabolism analytes or to alter a frequency of measurement of the creatinine related information or the optional additional one or more metabolism analytes.

The system may be configured for, based on said determining for the creatinine related information or one or more further optional metabolism analytes whether the obtained concentration information is within a predetermined range, providing the user with a warning for consulting a medical practitioner or for providing a warning to a medical practitioner of the user. The implantable sensor may be configured for simultaneously measuring a plurality of metabolism analytes in bodily tissue or bodily fluids of the living creature, thus obtaining a metabolic fingerprint for a living creature comprising concentration information of the plurality of metabolism analytes at any given moment in time.

The processor may be programmed for determining for each of the plurality of metabolism analyte whether the obtained concentration information is within a predetermined range.

The processor furthermore may be programmed for deriving from said determination a homeostatic and/or alleostatic condition of the living creature.

The plurality of metabolism analytes may comprise at least three different metabolism analytes. The plurality of metabolism analytes may be a selection of two or more of glucose, ketones, lactate, lactic acid, acetic acid, nitrite, creatinine, hormones, vitamine B11, vitamine B12, acetate, propionic acid, propionate, butyric acid, butyrate, phenyl sulfate, urea, pyruvate, ethanol, sugar, pH, uric acid, lactate dehydrogenase, fasting glucose, sodium, potassium or cholesterol.

The system may be adapted for obtaining information regarding the user's medical condition. The system furthermore may comprise an output port for providing an output to a user, a medical practitioner or a medical team and the health monitoring system may be programmed for providing personalised recommendations to the user through the output port.

The system may further comprise an input port for obtaining information regarding one or more physiological parameters of the living creature at the time of measurement of the creatinine related information or the one or more further optional metabolism analytes in bodily tissue or bodily fluids of the living creature.

The input port of the health monitoring system may be configured for receiving the information regarding one or more physiological parameters from a wearable, worn by the living creature. The system may be configured for receiving user input from a user, optionally through a graphical user interface.

The system may be programmed for prompting the user for input when one or more metabolism analyte concentrations outside the predetermined range is detected.

The system may be configured for deriving from the physiological parameters a stress condition and for identifying that the measured metabolism analyte concentrations are determined under the stress condition.

The system may comprise an input port for obtaining information regarding one or more of the user's location, hobbies, ethnic background, age, gender, socio-economical status, etc.

The processor may be configured for comparing with predetermined ranges for the metabolism analyte concentrations, whereby the predetermined ranges are based on previously measured creatinine related information or one or more further optional metabolism analyte concentrations from the living creature.

The processor may be configured for comparing with predetermined ranges for the metabolism analyte concentrations, whereby the predetermined ranges are based on previously measured creatinine related information or one or more further optional metabolism analyte concentrations obtained from a group of living creatures.

The system may be adapted for obtaining information regarding to any of gender, age, ethnicity or body mass index and wherein the processor furthermore is configured for comparing with predetermined ranges that are based on previously measured metabolism analyte concentrations obtained from a group of living creatures having a same gender, age, ....

The implantable sensor may comprise an optical sensor configured for spectroscopic measurement of the plurality of metabolism analytes in bodily tissue or bodily fluids.

The system furthermore may comprise an impedance spectroscopy sensor for deriving a hydration status.

In one aspect the present invention also relates to a health monitoring platform, the health monitoring platform comprising a plurality of health monitoring systems as described above for use by a plurality of individual users and further comprising a central processing unit for processing kidney-related parameters obtained from the plurality of health monitoring systems. Some embodiments of the invention provide a multiple user health monitoring system which comprises a plurality of the personal monitoring systems as described above. The multiple user health monitoring system comprises a remote server system which is provided for collecting the kidney related health profiles generated by the plurality of personal monitoring systems. As a result of the self-learning capabilities of the individual monitoring systems, the collected information can efficiently be used to generate e.g. reports, statistics, etc. of user groups.

In one aspect, the present invention also relates to a method of monitoring a kidney-related condition of a living creature, the method comprising
repetitively measuring creatinine information in bodily tissue or bodily fluids of the living creature and storing said creatinine information,
determining, from the stored information, a kidney-related parameter for the living creature. The method may comprise measuring, substantially simultaneously with the creatinine information, glucose related information in said bodily tissue or bodily fluids and storing said glucose related information. The kidney-related parameter may be determined from the combination of the stored creatinine information and glucose related information.

Measuring information may comprise obtaining spectrophotometric sensing data.

The method may comprise providing an output to a user, a medical practitioner or a medical team. Personalised recommendations regarding the kidney-related parameter may be provided to the user, e.g. through an output port.

The method may comprise comparing the creatinine information with a predetermined range, whereby the predetermined range is based on previously measured creatinine information from the living creature.

The method may comprise determining an estimated glomerular filtration rate (eGFR).

The method may take into account one, more or all of said at least one of age, length, weight, gender and/or ethnical characteristic of the human being for determining the estimated glomerular filtration rate.

The method may indicate a degree or stage of diabetes nephropaty and/or may indicate that a consult with a medical practitioner is advised.

The method may indicate, after a kidney transplant in a patient, a degree of acceptance of a transplanted kidney by the body of the patient.

The method may comprise time-dependent monitoring during a dialysis session and may indicate when a blood condition is sufficient to stop the dialysis session.

The method may trigger additional measurements of the creatinine related information or one or more optional additional metabolism analytes or may alter a frequency of measurement of the creatinine related information or the optional additional one or more metabolism analytes.

The method may, based on said determining for the creatinine related information or one or more further optional metabolism analytes whether the obtained concentration information is within a predetermined range, provide the user with a warning for consulting a medical practitioner or for providing a warning to a medical practitioner of the user.

The method may comprise simultaneously measuring a plurality of metabolism analytes in bodily tissue or bodily fluids of the living creature, thus obtaining a metabolic fingerprint for a living creature comprising concentration information of the plurality of metabolism analytes at any given moment in time.

The method may comprise using an algorithm to analyse the sensor data. The method may comprise detecting trends in sets of data points, and correlating trends of different sets of data points with each other. In this way, trends are detectable which are personal, i.e. specific to the user carrying the implanted sensor. Likewise, correlations between the trends in different biological parameters can be determined in a personal way, such as for example a normal evolution of creatinine and glucose concentration for that user or a normal evolution of creatinine and one or more other metabolite concentrations for that user. In this way, the method may be adapted for "learning" for example which are normal evolutions of the biological parameters of the user and which are not normal. The method may include this information in a personal profile which it generates for the user. This information can then be further used to for example make predictions, issue warnings, etc. In preferred embodiments according to the invention, the method may be configured for performing the following steps: determining first trends in creatinine concentration data points and second trends in glucose or other metabolite concentration data points; detecting first user dependent correlations between said first trends and said second trends, and generating a personal profile of the user based on said first user dependent correlations.

The method may comprise generating and/or communicating to the user personalized behavioural, life-style and therapeutic suggestions and actions, based on the monitoring.

Some embodiments of the invention provide a method for monitoring a kidney related parameter of at least one user. The method, and embodiments thereof, comprise substantially the steps as have already been described above in relation to the monitoring system according to the invention.

Similarly, the present invention also relates to a monitoring system comprising features for performing the method steps as expressed for a method or embodiments thereof as described above in relation to the monitoring method according to the invention.

In, another aspect, the present invention relates to a method of determining a kidney-related parameter of a living creature, the method comprising
receiving creatinine information repetitively measured in bodily tissue or bodily fluids of the living creature and storing the creatinine information, and
determining, from the stored information, a kidney-related parameter for the living creature.

The method may comprise, receiving glucose related information, substantially simultaneously measured with the creatinine information, in said bodily tissue or bodily fluids and storing the glucose parameter. The method may also comprise determining the kidney-related parameter from the combination of the stored creatinine information and the glucose related information. The information may comprise spectrophotometric sensing data. Determining a kidney-related parameter may comprise determining an estimated glomerular filtration rate (eGFR). Determining a kidney-related parameter may comprise taking into account one, more or all of said at least one of age, length, weight, gender and/or ethnical characteristic of the human being for determining the estimated glomerular filtration rate.

Particular and preferred aspects of the invention are set out in the accompanying independent and dependent claims. Features from the dependent claims may be combined with features of the independent claims and with features of other dependent claims as appropriate and not merely as explicitly set out in the claims.

These and other aspects of the invention will be apparent from and elucidated with reference to the embodiment(s) described hereinafter.

### Brief description of the drawings

The present invention will be discussed in more detail below, with reference to the attached drawings.
Fig. 1 shows an embodiment of the implantable sensor comprising sensor housing 1, sensing means 2, sensor housing adapted to sensing means 3 and a wireless transceiver 4.
Fig. 2 shows an embodiment of the implantable sensor with optical sensing means 5 and optical processor 6.
Fig. 3 shows an embodiment of the implantable sensor with components for wireless energy transfer 7.
Fig. 4 shows an embodiment of the implantable sensor with components for wireless energy transfer 7 and optical sensing means 5 and optical processor 6.
Fig. 5 shows an embodiment of the implantable sensor with processing means 9.
Fig. 6 shows an embodiment of the monitoring device comprising a wireless transceiver 4, monitoring device housing 8, processing means 9 and a memory 10.
Fig. 7 shows an embodiment of the monitoring device with display 11.
Fig. 8 shows an embodiment of the monitoring device with heart rate sensor 12.
Fig. 9 shows an embodiment of the monitoring device as an ensemble of devices.
Fig. 10 shows another embodiment of the monitoring device as an ensemble of devices.

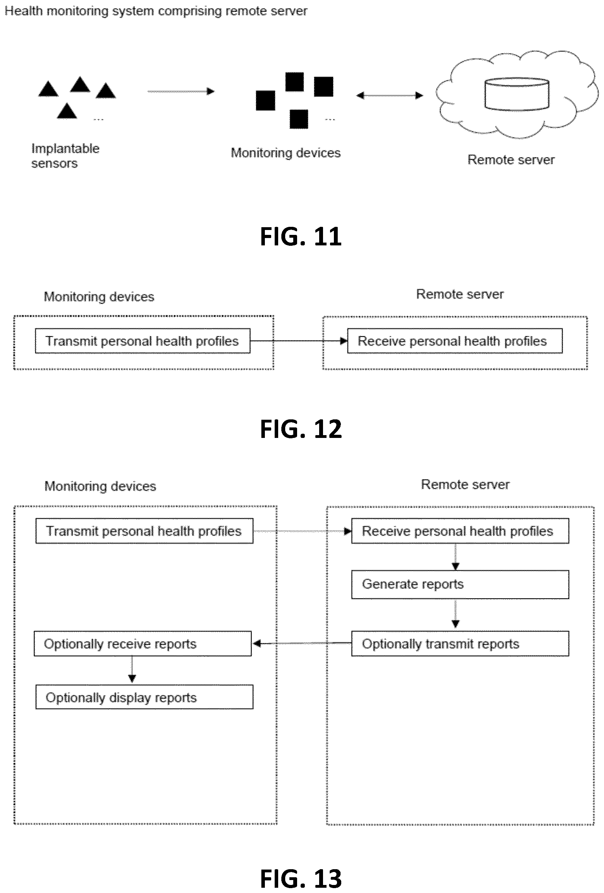

Fig. 11 shows an embodiment of the health monitoring system comprising a remote server.
Fig. 12 shows an embodiment of the interaction of monitoring devices with a remote server.
Fig. 13 shows another embodiment of the interaction of monitoring devices with a remote server.
Fig. 14 shows an embodiment of the interaction of the monitoring device with an insulin pump controller.
FIG. 15 shows a schematic overview of an embodiment of a health monitoring system according to an aspect of the present invention.

The drawings are only schematic and are non-limiting. In the drawings, the size of some of the elements may be exaggerated and not drawn on scale for illustrative purposes.

Any reference signs in the claims shall not be construed as limiting the scope.

In the different drawings, the same reference signs refer to the same or analogous elements.

### Detailed description of illustrative embodiments

The present invention will be described with respect to particular embodiments and with reference to certain drawings, but the invention is not limited thereto but only by the claims. The drawings described are only schematic and are non-limiting. In the drawings, the size of some of the elements may be exaggerated and not drawn on scale for illustrative purposes. The dimensions and the relative dimensions do not correspond to actual reductions to practice of the invention. Furthermore, the terms first, second and the like in the description and in the claims, are used for distinguishing between similar elements and not necessarily for describing a sequence, either temporally, spatially, in ranking or in any other manner. It is to be understood that the terms so used are interchangeable under appropriate circumstances and that the embodiments of the invention described herein are capable of operation in other sequences than described or illustrated herein.

Moreover, the terms top, under and the like in the description and the claims are used for descriptive purposes and not necessarily for describing relative positions. It is to be understood that the terms so used are interchangeable under appropriate circumstances and that the embodiments of the invention described herein are capable of operation in other orientations than described or illustrated herein.

It is to be noticed that the term "comprising", used in the claims, should not be interpreted as being restricted to the means listed thereafter; it does not exclude other elements or steps. It is thus to be interpreted as specifying the presence of the stated features, integers, steps or components as referred to, but does not preclude the presence or addition of one or more other features, integers, steps or components, or groups thereof. Thus, the scope of the expression "a device comprising means A and B" should not be limited to devices consisting only of components A and B. It means that with respect to the present invention, the only relevant components of the device are A and B.

Reference throughout this specification to "one embodiment" or "an embodiment" means that a particular feature, structure or characteristic described in connection with the embodiment is included in at least one embodiment of the present invention. Thus, appearances of the phrases "in one embodiment" or "in an embodiment" in various places throughout this specification are not necessarily all referring to the same embodiment but may. Furthermore, the particular features, structures or characteristics may be combined in any suitable manner, as would be apparent to one of ordinary skill in the art from this disclosure, in one or more embodiments.

Similarly, it should be appreciated that in the description of exemplary embodiments of the invention, various features of the invention are sometimes grouped together in a single embodiment, figure, or description thereof for the purpose of streamlining the disclosure and aiding in the understanding of one or more of the various inventive aspects. This method of disclosure, however, is not to be interpreted as reflecting an intention that the claimed invention requires more features than are expressly recited in each claim. Rather, as the following claims reflect, inventive aspects lie in less than all features of a single foregoing disclosed embodiment. Thus, the claims following the detailed description are hereby expressly incorporated into this detailed description, with each claim standing on its own as a separate embodiment of this invention.

Furthermore, while some embodiments described herein include some but not other features included in other embodiments, combinations of features of different embodiments are meant to be within the scope of the invention, and form different embodiments, as would be understood by those in the art. For example, in the following claims, any of the claimed embodiments can be used in any combination.

In the description provided herein, numerous specific details are set forth. However, it is understood that embodiments of the invention may be practiced without these specific details. In other instances, well-known methods, structures and techniques have not been shown in detail in order not to obscure an understanding of this description.

Where in embodiments of the present invention reference is made to a metabolic fingerprint, reference is made to concentration values of a set of metabolism analytes -as determined at a given moment in time. A metabolic fingerprint typically may comprise a plurality of metabolism analytes, such as for example two or more metabolism analytes or three or more metabolism analytes. Depending on the metabolism analytes enclosed in the metabolic fingerprint, the metabolic fingerprint may be dedicated for identifying information regarding particular diseases or conditions, or a set of diseases or conditions. In some embodiments of the present invention, the metabolic fingerprint may comprise a large number of metabolism analytes which may provide information regarding different conditions, disorders or diseases. It is an advantage of embodiments of the present invention that the metabolic fingerprint can easily be combined with temperature information.

Where in embodiments of the present invention reference is made to a homeostatic condition, reference is made to the tendency of having - at different states - a relative stable equilibrium of metabolism analytes. Where the indication "at different states" is given, reference is made to the fact that for example a stable equilibrium during the day can be different from a stable equilibrium during the night or that for example a stable equilibrium during summer can be different from a stable equilibrium during the winter.

Where in embodiments of the present invention reference is made to an alleostatic condition, reference is made to the tendency of maintaining - within a dynamic range - a state of internal, physiological equilibrium of metabolism analytes, in response to actual or perceived environmental and psychological stressors.

Where in embodiments of the present invention reference is made to an implantable sensor, reference is made to a sensor capable of in vivo measurements of one or more biological parameters in a living creature, e.g. in an animal or human. The implantable sensor may be located subcutaneous, intramuscular, intravascular, ocular such as in or attached to the cornea, in or attached to an organ, an oral space, a brain or a bone cavity. In an advantageous embodiment, the implantable sensor is a subcutaneous sensor.

In a first aspect, the present invention relates to a monitoring system for monitoring a kidney-related condition of a living creature. The monitoring system comprises an implantable sensor for repetitively measuring creatinine information in bodily tissue or bodily fluids of the living creature and for storing said creatinine information. It also comprises a processor programmed for determining, from the stored information, a kidney-related parameter for the living creature.

In embodiments of the present invention, the processor may be programmed for using calibration data, e.g. from calibration experiments relating the measured sensing data with a kidney-related parameter, using an algorithm or deriving correlation information based on artificial intelligence, for providing the kidney-related parameter based on the creatinine information measured in the bodily fluids or bodily tissues.

Embodiments of the present invention typically involve monitoring of creatinine related information, such as for example a creatinine concentration. Creatinine is a waste product produced by muscle metabolism. It is normally filtered out of the blood by the kidneys, but in patients with CKD, the kidneys are not able to do this effectively. Monitoring creatinine levels therefore can be used to estimate kidney function.

Creatinine levels in the blood are in the state of the art typically measured by a laboratory test, which requires a blood sample to be taken and sent to a laboratory for analysis.

It is an advantage of embodiments of the present invention that continuously monitoring of creatinine levels can be performed which is a useful tool for monitoring the progression of CKD. By tracking changes in creatinine levels over time, physicians can for example make more informed decisions regarding the best course of treatment for the patient.

In some embodiments, a slope of decline in kidney function can be monitored. In addition, continuous monitoring of creatinine levels can provide early detection of changes in kidney function, allowing for earlier interventions and potentially slowing the progression of CKD. Continuous measurement of creatinine is in the state of the art not a routine practice in CKD management and requires specialized technology and expertise.

As mentioned before, in the state of the art creatinine levels are typically measured through blood tests, which are usually done periodically, often every three to six months, depending on the stage of the disease. These periodic measurements may not provide a complete picture of a patient's kidney function, as the level of creatinine can change rapidly in response to changes in the patient's health or medications.

According to embodiments of the present invention, a continuous monitoring device for creatinine is provided allowing providing real-time information on kidney function and helping healthcare providers adjusting treatments and medications as needed. This could improve patient outcomes and potentially reduce healthcare costs by allowing earlier interventions to prevent or slow CKD progression.

The kidney may be one of an artificial kidney, a transplanted kidney or an own kidney of the living creature.

It is an advantage of embodiments of the present invention that the frequency at which creatinine or other metabolites are measured can be altered as function of a stage of the disease and/or as function of the individual patient's circumstances.

It is an advantage of embodiments of the present invention that creatinine related information can be measured in real-time or continuously throughout the day.

According to embodiments of the present invention, the implantable sensor is configured for substantially simultaneously measuring glucose related information in said bodily tissue or bodily fluids and for storing said glucose related information, and the processor is programmed for determining a kidney-related parameter from the combination of the stored creatinine information and glucose related information.

In some embodiments, the implantable sensor is configured for obtaining spectrophotometric sensing data and for deriving the creatinine information and the glucose related information from the same spectrophotometric sensing data. In some embodiments, creatinine is measured in interstitial fluid using a spectrophotometric technique such as measurement in near infrared. According to some embodiments, the monitoring system furthermore comprises an output port for providing an output to a user, a medical practitioner or a medical team and wherein the monitoring system is programmed for providing personalised recommendations regarding the kidney-related parameter to the user through the output port.

The creatinine information may be a creatinine concentration and the processor may be configured for indicating whether the obtained concentration information is within a predetermined range, for providing the user with a warning for consulting a medical practitioner or for providing a warning to a medical practitioner of the user.

The processor may be configured for comparing the creatinine information with a predetermined range, whereby the predetermined range is based on previously measured creatinine information from the living creature.

The processor may be adapted for determining an estimated glomerular filtration rate (eGFR). In some embodiments, the system furthermore comprises an input port configured for receiving information regarding at least one of an age, length, weight, gender and/or ethnical characteristic of the human being and the processor being configured for taking into account one, more or all of said at least one of age, length, weight, gender and/or ethnical characteristic of the human being for determining the estimated glomerular filtration rate.

The monitoring system may be adapted for indicating a degree or stage of diabetes nephropaty or Lupus Nephritis and/or for indicating that a consult with a medical practitioner is advised.

In some embodiments, the system is configured for indicating a stage of chronic kidney disease. The different stages of CKD are based on the level of kidney function, which is typically determined by the glomerular filtration rate (GFR). GFR is a measure of how much blood passes through the kidneys each minute, and it is calculated using a formula that considers a person's age, sex, and serum creatinine level, the formula being known in the art. The GFR is used to check the kidney function.

The stages of CKD are as follows:
Stage 1: Kidney damage with normal or increased GFR (GFR > 90 ml/min)
Stage 2: Mildly decreased GFR (GFR = 60-89 ml/min)
Stage 3: Moderately decreased GFR (GFR = 30-59 ml/min)
Stage 4: Severely decreased GFR (GFR = 15-29 ml/min)
Stage 5: Kidney failure (GFR < 15 ml/min or on dialysis)

According to embodiments of the present invention, using calibration data for example using known characterisation methods for creatinine levels, spectrophotometric creatinine data can be correlated with the creatinine levels, thus allowing to derive an estimate of the creatinine clearance or an estimate of the GFR for a patient.

Each stage represents a progressively more severe loss of kidney function, with Stage 5 representing end-stage renal disease (ESRD) where the kidneys have lost almost all of their function. It is an advantage of embodiments of the present invention that early detection and treatment of CKD can be obtained, allowing to slow down or even halt its progression. It is an advantage that accurate monitoring can Abe performed, since the implantable sensor allows for easy repetitive measurement of relevant parameters, e.g. continuously, stepwise, time-dependent, algorithm based measuring.

The monitoring system may be adapted for detection of a worsening renal function and/or for prognosis of the renal function of the patient.

According to some embodiments, the implantable sensor is furthermore configured for detecting one or more of lipids, urea or albumin and the processor is programmed for using the creatinine information in combination with the one or more of lipids, urea or albumin for monitoring chronic kidney disease progression.

In particular embodiments one or more of the following metabolites thus may be monitored: Blood urea nitrogen (BUN) : BUN is a waste product that is produced when the body breaks down proteins. Like creatinine, it is normally filtered out of the blood by the kidneys. Elevated BUN levels in patients with CKD can indicate reduced kidney functions

Albumin: Albumin is a protein that is produced by the liver and is found in the blood. In patients with CKD, low levels of albumin can indicate malnutrition, inflammation, or liver disease In some embodiments, the system also may be equipped with a measurement system, e.g. additional sensor, for measuring electrolytes, such as potassium or minerals such as phosphorus and calcium.

In some embodiments, the monitoring system is adapted for indicating, after a kidney transplant in a patient, a degree of acceptance of a transplanted kidney by the body of the patient and/or monitoring a graft stability. A particular output with respect to the degree of acceptance of the transplanted kidney by the body of the patient and/or with respect to the graft stability may be provided.

In some embodiments, the system is configured for time-dependent monitoring during a dialysis session and the processor is configured for indicating a blood condition as function of the dialysis that is performed in the dialysis session. The processor may be configured for outputting an indication that, once a blood condition is determined to be appropriate, the dialysis session may be stopped. The system may be adapted for capturing at least creatinine information.

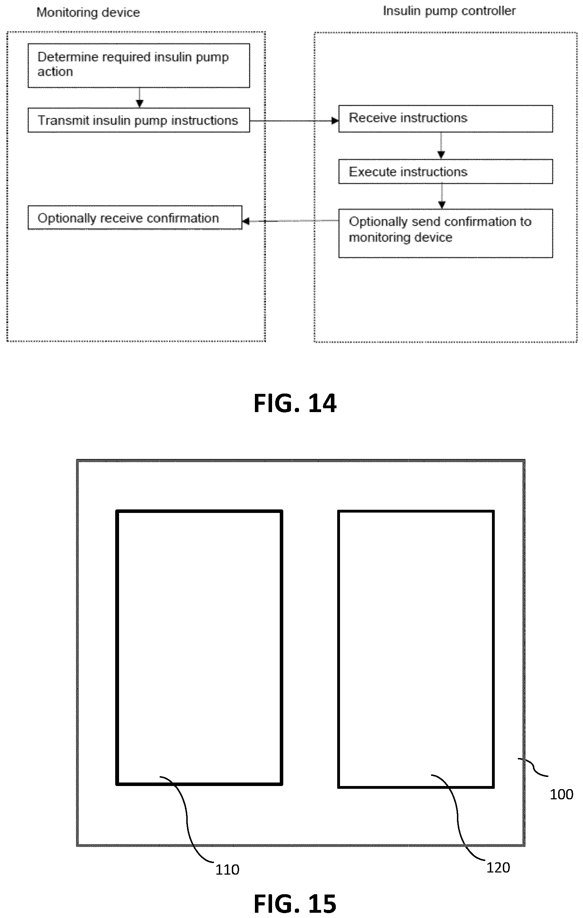

An exemplary embodiment of a system according to an embodiment of the present invention is shown in FIG. 15. The health monitoring system 100 comprises an implantable sensor 110 for measuring creatinine information and optionally also one or more other metabolites at any moment in time, and a processor 120 programmed for determining therefrom a kidney-related parameter.

By way of illustration, embodiments of the present invention not being limited thereby, standard and optional components of the monitoring system are described in more detail.

According to embodiments of the present invention, the monitoring system comprises an implantable sensor that is configured for measuring creatinine information and optionally also one or more other metabolites at any moment in time. The monitoring system may be a system for monitoring health of a living creature by performing measurements continuously, at predetermined moments in time, at moments in time triggered by events such as for example based on an environmental and/or lifestyle driven input such as for example driven by food intake or any other incoming information such as for example sound, tactile, smell, heat or movement. Measurements may be performed at any desired moment in time, etc. It is an advantage of embodiments of the present invention that there is no need for manual intervention, i.e. measurements can be performed in an automated way and automatically, due to the fact that measurements are based on an implantable sensor. It is an advantage of embodiments of the present invention that monitoring can be performed without the need for consulting a medical practitioner at regular moment in times.

It is an advantage of embodiments of the present invention that measurements are performed in situ / in vivo, allowing for measuring and monitoring in the real environment, i.e. without the need for determining values of e.g. metabolism analytes on samples that are first extracted from the living creature. Extraction from the living creature typically may result in alteration of the samples and therefore may result in deviations of the measured metabolism analytes compared to their in vivo presence.

The implantable sensor of the health monitoring system according to an exemplary embodiment of the present invention typically may comprise a sensor housing, a sensing device for sensing creatinine information in bodily tissue or bodily fluids and optionally one or more further metabolites. The implantable sensor also typically may comprise a wireless transceiver for transmitting sensor data containing data points which are provided by said sensing means upon sensing biological parameters. The implantable sensor may be capable of performing a reagent-free analysis method. In some embodiments, the implantable sensor may comprise biocompatible packaging in order to avoid and/or minimize the risk of bio-fouling. The implantable sensor may comprise a battery, a rechargeable battery, capacitors and/or components for wireless energy transfer. The system may be equipped with components for wireless energy transfer. In particular embodiments, the system may be equipped with a rechargeable battery such as for example with a solid state battery.

The implantable sensor may comprise an optical sensing device configured for spectroscopic measurement of the creatinine information and optionally one or more plurality of metabolism analytes in bodily tissue or bodily fluids. It is an advantage of embodiments that spectroscopic measurements allows for detection of creatinine and optionally one ore more further metabolism analytes, e.g. based on detection of absorption in different absorption bands.

It is an advantage of embodiments of the present invention that optical spectroscopy based on an implantable sensor allows for accurate determination of metabolite concentrations. The implantable sensor may for example be a semiconductor-based photonics integrated circuit sensor, such as for example a silicon-based photonics integrated circuit sensor as for example described in European patent application EP10760633.7 although embodiments are not limited thereto. It is an advantage of embodiments of the present invention that a miniaturised sensing device can be used that is at the same time small in footprint, thus providing good implantability conditions, and allows for accurate concentration determination of metabolites. It is an advantage of embodiments of the present invention that optical spectroscopy of metabolites in bodily tissue or bodily fluids based on an implantable sensor allows for detection of small concentration variations, thus resulting in accurate determination of metabolite concentration information.

In an advantageous embodiment, the sensing device also may allow for determining body temperature from the measurement. Alternatively, or in addition thereto a dedicated temperature sensing device may be included in the implantable sensor.

It is an advantage of embodiments of the present invention that measurements can be performed at very short timescale, thus allowing detection of metabolism analyte concentration variations at very short timescale, such as for example on a timescale of less than 5 minutes, e.g. in some embodiments a timescale of less than one minute, e.g. in some embodiments within seconds or parts of seconds.

It is an advantage of embodiments of the present invention that also middle- and long-term variations can be identified, e.g. variations on a timescale of hours, days or weeks. Furthermore, some variations may be time-of-year dependent, and the health monitoring system also may identify such variations, e.g. by comparison of results month to month.

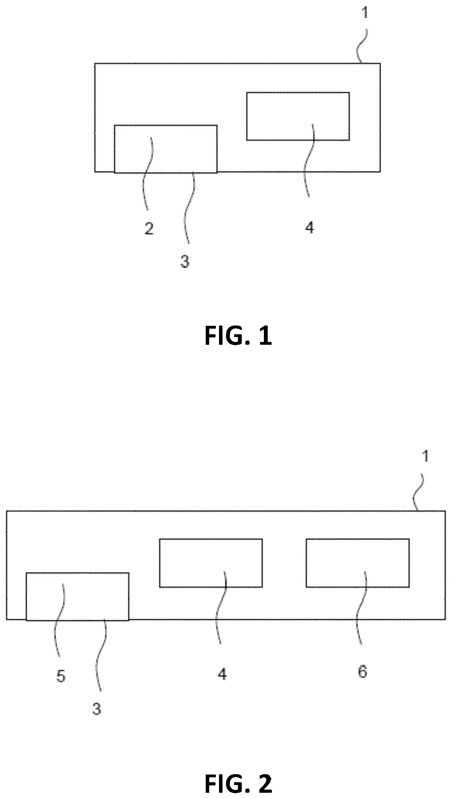

According to some embodiments, the implantable sensor may correspond with an implantable sensor as shown and described in FIG. 1 to FIG. 5, but whereby the implantable sensor is particularly adapted for obtaining creatinine information and optionally one or more further metabolites.

The system furthermore may comprise an impedance spectroscopy sensing device for deriving a hydration status. The system may be configured for measuring a physiological ionic status. Based on impedance spectroscopy, changes in the physiological ionic status in interstitial fluids can be determined, allowing evaluation of a hydration status of a user. Impedance spectroscopy analysis thereby may be based on e.g. Nyquist/Warburg plots of the derived information.

In some embodiments, also other types of sensing devices may be included in the implantable sensor such as for example an electrochemical sensing device, an enzymatic sensing device, an electronic impedance sensing device, a resonant electronic circuit sensing device, or an electrical sensing device such as a resistive sensing device. Also, other type of sensing devices may be included such as for example accelerometers, orientation sensing devices, pH sensing devices, electrical activity (e.g. conductivity) sensing devices, etc. It is an advantage of embodiments of the present invention that different metabolites can be determined simultaneously based on detection in bodily tissue or fluids, thus allowing a more accurate evaluation of a unique health condition of a living creature. Whereas often statistically driven information may be used for controlling and/or monitoring health, in practice effects may in principle be personal and therefore unique to certain living creatures. It is an advantage of embodiments of the present invention that it allows identifying whether effects - which statistically may occur for a population - are present for the individual or that it allows identifying the conditions under which such effects occur for an individual. It thus is an advantage of embodiments of the present invention that a real personalised health monitoring system is obtained.

It is an advantage of embodiments of the present invention that the health monitoring system can be used as a tool for investigating whether certain effects occur for individuals or for given populations and for investigating for example the effect of food, lifestyle choices or drug uptake on the individual or on a given population.

It is an advantage of embodiments of the present invention that mutual influences or combined effects can be accurately determined since the obtained concentration information stems guaranteed from the same moment in time, i.e. embodiments allow for obtaining paired data. The obtained data does not need to comprise or require contextual data, although embodiments of the present invention are not limited thereto and, as will be described below, contextual data may be provided, e.g. from manual user input, through application programs on computerised devices of the user or in any other way.

According to embodiments of the present invention, the monitoring system also may comprise a processor programmed for determining a kidney related parameter. The processor may be programmed for determining whether the creatinine information and the optional one or more further metabolism analytes whether the obtained concentration information is within a predetermined range. The processor may be any suitable type of processor for processing data as known in the field, such as for example a general-purpose processor or a specific processor, but according to embodiments of the present invention, the processor is programmed for determining for each of the plurality of metabolism analyte whether the obtained concentration information is within a predetermined range. Such ranges may be personalised ranges determined for the individual, may be ranges based on statistical information for specific groups of users, may be ranges determined based on particular physiological parameters of the users, and alike, as will be further described below.

The processor can be for example implemented on the implantable sensor sending the processed data wireless to an external device. Such an external device may be a dedicated device or a mobile device, such as for example a smartphone, a watch, a tablet or a laptop, or it may for example be a computer or may be a cloud-based storage device. Alternatively, the processor can also be implemented or part of an external device such as for example a dedicated device or a mobile device, such as for example a smartphone, a watch, a tablet or a laptop, a computer or a cloud-based storage device. Such an external device typically also comprises a wireless transceiver for receiving the raw or processed data. Data communication between the sensing device and the processor may be performed based on any suitable protocol. Data communication between the implantable sensor and the external device may be established using any suitable protocol, as known in the field.

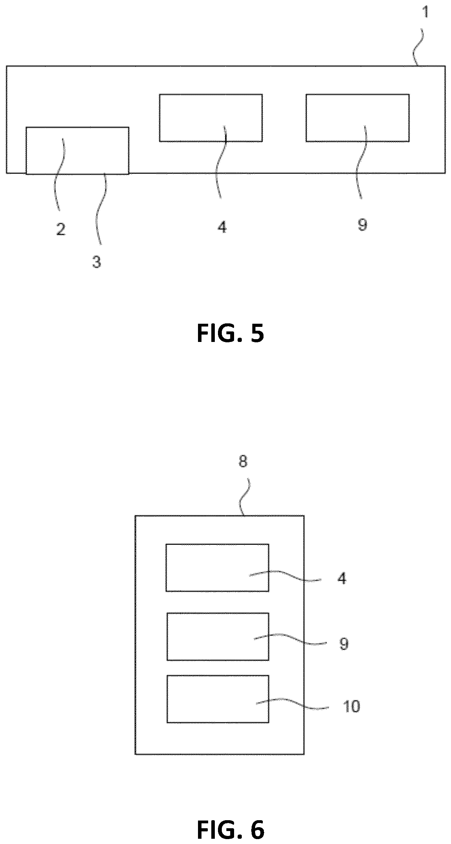

By way of illustration, embodiments of the present invention not being limited thereto, external devices as can be used in embodiments of the present aspect may be as shown and described in FIG. 6 to FIG. 10, but wherein the external device is arranged for obtaining processed data comprises information regarding whether the metabolism analyte concentration is within predetermined ranges, as indicated above, or wherein a processor is present on the external device is programmed for such processing.

The processing also may be split over different processors which may be implemented on the implantable device, on an external device or splitted over the implantable sensor and the external device. The processing may in some embodiments be splitted into cloud-base processing.

The processing may be based on a predetermined algorithm. In some embodiments, processing may make use of artificial intelligence, neural networks, etc.

The processor may be configured for comparing with predetermined ranges for the metabolism analyte concentration, whereby the predetermined ranges are based on previously measured metabolism analyte concentrations from the living creature.

The predetermined range thus can be uniquely identified for each user. Again in this way, a health monitoring system is provided that really is designed for the individual using the system.

The processor may be configured for comparing with a predetermined range for the metabolism analyte concentration, whereby the predetermined range is based on previously measured metabolism analyte concentrations obtained from a group of living creatures.

The predetermined range can in some embodiments be based on statistically-pooled health indications.

The system may be adapted for obtaining information regarding to, for example, any of gender, age, ethnicity or body mass index and wherein the processor furthermore is configured for comparing with predetermined ranges that are based on previously measured metabolism analyte concentrations obtained from a group of living creatures having a same gender, age, ....

It is an advantage of embodiments of the present invention that the monitoring may allow for identifying risks to certain conditions or diseases or to better manage conditions or diseases e.g. by providing recommendations for controlling lifestyle, dietary conditions or controlling personal drug uptake. It also may be used for identifying a personalised and unique response to dietary conditions, lifestyle decisions or personal drug uptake.

In some embodiments, aside the creatinine information, two or more metabolism analytes may be measured and determined. It is an advantage of embodiments of the present invention that information regarding more than two, e.g. three or more metabolism analytes can be determined, thus allowing to provide good information regarding a health condition of the living creature, e.g. of a homeostatic and/or alleostatic condition of the living creature homeostatic and/or alleostatic condition of the living creature. The latter may allow for co-monitoring creatinine with other relevant analytes.

The one or more further metabolism analytes may be a selection of one, two or more of glucose, ketones, lactate, lactic acid, hormones, acetic acid, nitrite, vitamine B11, vitamine B12, acetate, propionic acid, propionate, butyric acid, butyrate, phenyl sulfate, urea, pyruvate, ethanol, sugar, pH, uric acid, lactate dehydrogenase, fasting glucose, sodium, potassium or cholesterol. Water and temperature also may be used as biological parameters for monitoring health. The health monitoring system may for example be adapted for identifying two or more metabolites of the group of lactate, ketones, ethanol, sugar or pH. The health monitoring system may use such additional analytes in combination with creatinine to obtain a metabolic fingerprint for deriving information regarding risks for chronic kidney disease or metabolic acidosis by a living creature. According to embodiments of the present invention, the monitoring system may be adapted for obtaining information regarding the user's medical condition. The medical condition may comprise information obtained from an electronic patient file, obtained directly from the user, obtained from a medical practitioner, obtained based on medical interventions, obtained from medical examination, etc.

Information from health monitoring systems of different users may be shared at a central server, optionally anonymised. It is an advantage of embodiments of the present invention that such a system may allow for gathering information from a large group of users. Based on the information obtained, big data analysis may be performed and correlations between certain measured data and medical conditions or certain pathologies may be identified, which can be used for future health care optimisation. It is an advantage of embodiments of the present invention that in big data analysis predetermined algorithms, artificial intelligence including neural networks, self-learning algorithms and alike may be used for identifying such correlations.

The system may be configured to measure creatinine and optional further different metabolism analytes at different moments in time.

The health monitoring system may be adapted for measuring the creatinine and optionally one or more metabolism analytes continuously, quasi-continuously or at predetermined intervals over time.

The system may be configured to, based on said determining for the creatinine information and optionally one or more metabolism analytes whether the obtained concentration information is within a predetermined range, trigger additional measurements of one or more metabolism analytes or to alter a frequency of measurement of one or more metabolism analytes.

It is an advantage of embodiments of the present invention that dynamic adjustment of the monitoring can be performed, thus resulting in a more personalised monitoring and optionally continuous monitoring and thus allowing better control and management of the health for a specific user.

According to embodiments of the present invention, the system may be configured for, based on said determining for creatinine and optional the one or more further metabolism analytes whether the obtained concentration information is within a predetermined range, providing an output to a user, to a medical practitioner, to a medical team or to other interested parties. The health monitoring device therefore may comprise an output means, e.g. output port, which may for example be implemented in the external device, e.g. in a dedicated device or in a dedicated computer program application. Based on the processed data, personalised recommendations can be made for potential interventions for driving and improving the health of the user. Such personalised recommendations may be based on collected personal and/or global information. Output may include providing the user with a warning for consulting a medical practitioner or for providing a warning to a medical practitioner of the user, with providing timing information for scheduling a further consult with a medical practitioner, advising certain medical examination, ... The output also may comprise merely data information regarding the obtained concentration information of the plurality of metabolism analytes, for displaying warnings regarding the measured metabolism analytes, for indicating deviations of the measured metabolism analyte with respect to a predetermined range, and alike.

The monitoring system may be equipped with an auditive or display system for displaying information as identified above.

According to embodiments of the present invention, the monitoring system further may comprise an input port for obtaining information regarding one or more physiological parameters of the living creature at the time of measurement of the plurality of metabolism analytes in bodily tissue or bodily fluids of the living creature.

The physiological parameter may be one or more of a temperature, information regarding the heart rate such as for example a heart rate variability, blood pressure, motion-based activity, food uptake, tissue conductivity, brain activity such as for example alpha, beta, gamma, delta-wave patterns, respiratory rate, oxygen or carbon monoxide saturations, physical activity, sleep pattern, menstruation cycle, stress, agenda, etc.

In some embodiments, the system may be adapted for obtaining the input from a user by manual input, auditive input, tactic input, .... The system may be equipped with a graphical user interface. The input port of the health monitoring system may be configured for receiving the information regarding one or more physiological parameters from a wearable, worn by the living creature. Such a wearable may comprise a temperature sensor, a heart pulse rate sensor, a motion sensor, a sensor configured for identifying food uptake, a blood pressure sensor, temperature, information regarding the heart rate such as for example a heart rate variability, blood pressure, motion-based activity, food uptake, tissue conductivity, brain activity such as for example alpha, beta, gamma, delta-wave patterns, respiratory rate, oxygen or carbon monoxide saturations, physical activity, sleep pattern, agenda, position, weather conditions, etc ...

It is an advantage of embodiments of the present invention that a good synchronisation between the measured metabolism analyte concentration information and the physiological parameter information is obtained, thus allowing more accurate correlation between measured metabolism analyte concentration information and events such as food uptake, stress, activity, and alike. The monitoring system may be configured for receiving user input. The system may be adapted for receiving user input, e.g. manual user input, such as information regarding a food uptake event which may for example be combined with information regarding a blood sugar level, information regarding any of the above identified activities or conditions.

The monitoring system may be adapted for prompting the user for input when one or more metabolism analyte concentrations outside the predetermined range is detected.

It is an advantage of embodiments of the present invention that user input may be prompted for when a detection of a metabolism change is performed by the system. Since the monitoring system allows for continuous monitoring, upon detection of a change, direct information from the user can be obtained, allowing better corelation between a metabolism change and e.g. an activity or event.

The monitoring system may be adapted for receiving information regarding the user via user input, via apps used by the user, ....

The monitoring system may be configured for deriving from the physiological parameters a stress condition and for identifying that the measured metabolism analyte concentration is measured under the stress condition. The system may furthermore be triggered for performing further measurement of metabolism analytes at a later moment in time.

It is known that stress influences metabolism through activation and deactivation of specific functions as controlled by the autonomous nervous system during sympathetic or parasympathetic activation. This has an impact on a number of metabolism analytes in the body of a living creature (e.g. whether digestion takes place at its full functionality or not). Concentration information of metabolism analytes obtained under certain stress conditions therefore may provide an image of the metabolic response during a stressful (sympathetic activation) situation and give information to the metabolic response under a given autonomic activation (sympathetic = stress = fight or flight, parasympathetic = rest & digest). This information can provide input to the actual health condition of the living creature and can trigger appropriate intervention to improve it (e.g. by proactively assisting in parasympathetic activation previous and/or during food ingestion, for example through guided exercises such as breathing). It is an advantage of embodiments of the present invention that a health monitoring system based on an implantable sensor allows for obtaining information at different moment in times. Whereas conventional analysis of metabolism analytes is typically based on sampling e.g. blood from a living creature which typically needs to be done by a medical practitioner and which is an action known to induce stress for several patients, the continuous monitoring system of embodiments of the present invention can obtain information at any moment in time, without - once the implantable sensor has been implanted - the need for intervention of a medical practitioner. Therefore, the monitoring system allows for the possibility of obtaining more trustworthy metabolism analyte concentration information obtained at a moment in time where the living creature is less subject to the measurement stress and where the impact of direct environmental factors (e.g. stressful environment) can be detected. The latter allows for a more accurate determination of the actual personalized response of the metabolism and thus the health condition of the living creature. The monitoring system may comprise an input port for obtaining information regarding one or more of the user's location, hobbies, ethnic background, age, gender, socio-economical status, etc.

The health monitoring system may be adapted for receiving information regarding the user via user input, via apps used by the user, ....

Information from monitoring systems of different users may be shared at a central server, optionally anonymised.

According to one aspect, the present invention also relates to a health monitoring platform, the health monitoring platform comprising a plurality of health monitoring systems as described above for use by a plurality of individual users and further comprising a central processing unit for processing metabolic fingerprint information obtained from the plurality of health monitoring systems. The central processing unit may for example be a remote server system, which is arranged for collecting the metabolic fingerprint information from different users or personal health information from different users, obtained through the plurality of health monitoring systems. In another embodiment, the platform is equipped with an algorithm for deriving information regarding metabolic fingerprints and certain conditions or diseases, for deriving information regarding physiological parameters and metabolic fingerprints, for storing and providing recommendations based on certain measured metabolic fingerprints optionally completed with other information, and alike. The platform may be organised whereby the data information provided by the individual health monitoring systems comprises metabolic fingerprints and information regarding whether the metabolism analyte concentrations are within predetermined ranges.

It is an advantage of embodiments of the present invention that such a system may allow for gathering information from a large group of users. Based on the information obtained, big data analysis may be performed and correlations between certain metabolism analyte fingerprints and other information such as for example physiological information or other user information. The latter may assist in identifying causes of certain metabolism-based diseases. The latter may for example also be used for specific targeted investigation trials in chich the influence of multiple parameters can be studied.

It is an advantage of embodiments of the present invention that predetermined algorithms, artificial intelligence including neural networks, self-learning algorithms and alike may be used for identifying such correlations. It is an advantage of embodiments of the present invention that in big data analysis predetermined algorithms, artificial intelligence including neural networks, self-learning algorithms and alike may be used for identifying such correlations.

In one aspect, the present invention also relates to methods formonitoring a kidney-related condition of a living creature. The method comprises repetitively measuring creatinine information in bodily tissue or bodily fluids of the living creature and storing said creatinine information. The method also comprises determining, from the stored information, a kidney-related parameter for the living creature.

According to some embodiments, a method for obtaining personal information for an individual is disclosed, the method comprising measuring creatinine related information and one or more further metabolism analytes in bodily tissue or bodily fluids of the living creature. The method may comprise determining a creatinine concentration and determining whether the obtained concentration information is within a predetermined range. In some embodiments, the method may comprise providing an output to a user, a medical practitioner or a medical team, the output comprising personalised recommendations. The monitoring system as described in embodiments of the present invention may be programmed for performing the method steps as described above, e.g. programmed with a corresponding algorithm. In some embodiments, the method comprises substantially simultaneously measuring glucose related information in said bodily tissue or bodily fluids and for storing said glucose related information, wherein the processor is programmed for determining a kidney-related parameter from the combination of the stored creatinine information and glucose related information.

In some embodiments, the method comprises indicating, after a kidney transplant in a patient, a degree of acceptance of a transplanted kidney by the body of the patient and/or monitoring a graft stability.

In some embodiments, the method comprises time-dependent monitoring during a dialysis session and indicating a blood condition as function of the dialysis that is performed in the dialysis session.

Illustrative examples are provided herein to demonstrate principles and advantages of embodiments according to the present invention. These examples are not intended to be limiting to the invention in any way, but merely provided to assist the skilled person in reducing embodiments of the present invention to practice.

In, another aspect, the present invention relates to a method of determining a kidney-related parameter of a living creature. The method thereby comprises receiving creatinine information repetitively measured in bodily tissue or bodily fluids of the living creature and storing the creatinine information, and determining, from the stored information, a kidney-related parameter for the living creature. The method may comprise, receiving glucose related information, substantially simultaneously measured with the creatinine information, in said bodily tissue or bodily fluids and storing the glucose parameter. The method may also comprise determining the kidney-related parameter from the combination of the stored creatinine information and the glucose related information. The information may comprise spectrophotometric sensing data.

Determining a kidney-related parameter may comprise determining an estimated glomerular filtration rate (eGFR). Determining a kidney-related parameter may comprise taking into account one, more or all of said at least one of age, length, weight, gender and/or ethnical characteristic of the human being for determining the estimated glomerular filtration rate.

The implantable sensor of the health monitoring system of the present invention is shown in Fig. 1 and comprises a sensor housing 1, a means for sensing biological parameters in bodily fluids 2, an area of the sensor housing which is adapted to the sensing means 3 and a wireless transceiver 4. The implantable sensor may be capable of sensing biological parameters based on surface chemical reactions and/or by using optical means. The implantable sensor may be capable of performing a reagent-free optical analysis method. The implantable sensor may comprise biocompatible packaging in order to reduce or minimize the risk of bio-fouling.

A preferred embodiment of an implantable sensor is shown in Fig. 2 and is equipped with optical sensing means 5 and an optical processor 6 (e.g. a single-chip optical sensor) for continuous analyte monitoring. The implantable sensor comprises an advanced optical processor in the sensor, allowing advanced and optionally complex radiation processing, e.g. allowing spectral and depth-resolved processing of radiation received or guided to a measurement region.

The implantable sensor comprises means for sensing creatinine in bodily fluids and comprises a wireless transceiver for transmitting sensor data containing data points which are provided by said sensing means upon sensing the creatinine. The implantable sensor is provided for measuring at least creatinine in bodily fluids.

In embodiments of the invention, the implantable sensor may be adapted for sensing at least creatinine in bodily fluids wherein the bodily fluid may be interstitial fluid, ocular fluid, intermuscular fluid or peritoneal fluid. It has been found that measurements of creatinine in the interstitial fluid presents a reliable relationship with blood values, is minimally invasive and safe and presents other advantages such as the elimination of the need for anticoagulants. Thus, in a preferred embodiment of the invention, the implantable sensor is a subcutaneous implantable sensor.

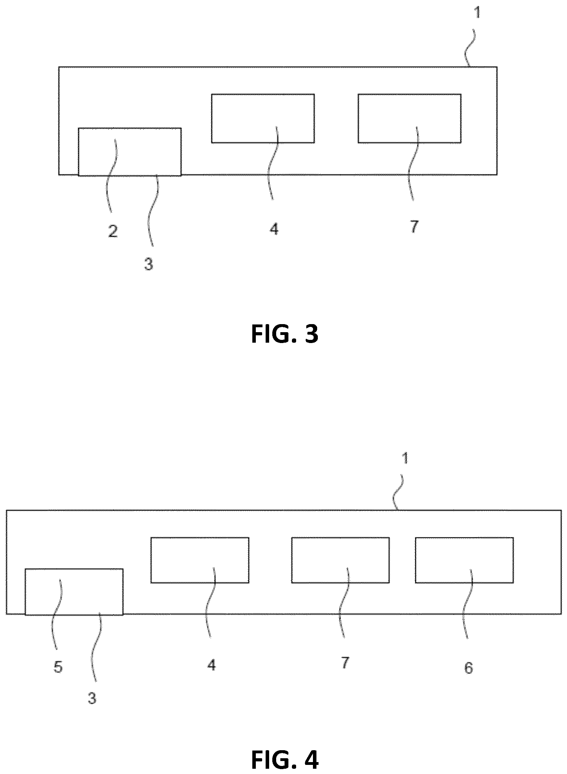

In embodiments of the invention, the implantable sensor may comprise a rechargeable battery and/or components for wireless energy transfer. A preferred embodiment of an implantable sensor is shown in Fig. 3 and is equipped with components for wireless energy transfer 7. A more preferred embodiment of an implantable sensor is shown in Fig. 4 and is equipped with optical sensing means 5 and an optical processor 6 and components for wireless energy transfer 7. In a preferred embodiment, the rechargeable battery is a solid state battery.

In embodiments of the invention, the implantable sensor may further be equipped with means for processing sensor data, wherein said processing means is equipped with an algorithm which is executable on said processing means and which is provided for converting sensor data before transmitting to the monitoring device. A preferred embodiment of an implantable sensor is shown in Fig. 5 and is equipped with a processing means 9.

The implantable sensor may further be capable of sensing heart rate, body temperature, urea, lactate, pH, fructosamine, oxaloacetate and/or hydration level.

A preferred embodiment of a monitoring device is shown in Fig. 6 and comprises a housing 8, a wireless transceiver 4 for communicating with the wireless transceiver of the implantable sensor or other devices of the monitoring device to receive sensor data. The monitoring device further comprises processing means 9 for processing said sensor data and a memory 10 for storing data. The processing means is equipped with an algorithm, which is loadable into the memory 10 for execution by said processing means.

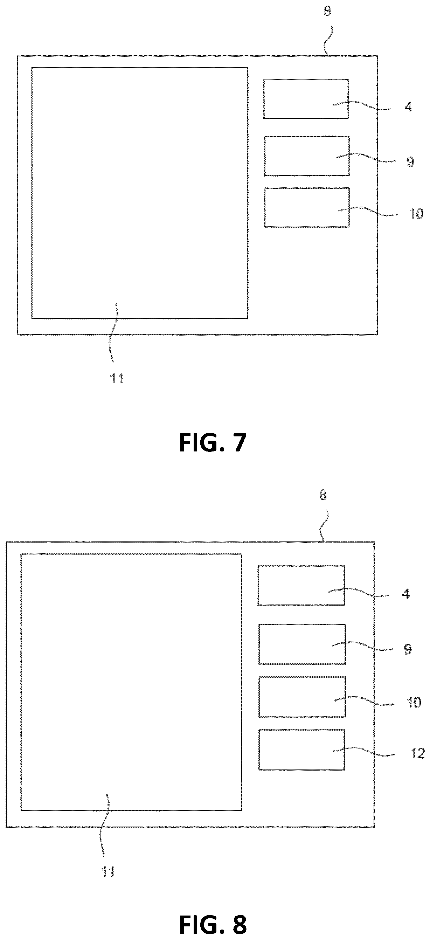

Another embodiment of the monitoring device is shown in Figure 7 wherein the monitoring device is equipped with a user interface, comprising a display 11 for displaying the personal health profile and interacting with the user. The monitoring device may further be provided for receiving heart rate data points from a heart rate sensor, said heart rate data points being received from the implantable sensor or any other device which is capable of providing heart rate data points. For example, in case the monitoring device is a smart watch, a heart rate sensor may be provided on board the smart watch. Figure 8 shows a preferred embodiment of the monitoring device wherein the monitoring device is equipped with a heart rate sensor 12.

In another embodiment of the present invention the monitoring device may further be provided for receiving and processing data points of one or more of the following parameters: body temperature, urea, lactate, pH, fructosamine, oxaloacetate and/or hydration level. These parameters may be provided by the implantable sensor or any one or more additional implantable sensors and/or other device which is capable of providing data points of one or more of said parameters. The algorithm executable on the processing means may then be provided for determining trends in the data points of the one or more additional parameters (body temperature, urea, lactate, pH, fructosamine, oxaloacetate and/or hydration level) and generating a personal health profile. In a preferred embodiment the algorithm executable on the processing means may then be provided for determining trends in the data points of the one or more additional parameters (body temperature, urea, lactate, pH, fructosamine, oxaloacetate and/or hydration level), detecting user dependent correlations between said trends and trends in heart rate data points, trends in creatinine concentration data points and optionally glucose concentration data points, and evaluating said user dependent correlations upon generating a personal health profile. Thus, a further improved personal health profile may be achieved.

In another embodiment of the present invention the monitoring device may further be provided for receiving and processing data points of one or more of the following parameters: nutritional intake such as carbohydrate intake data points, activity such as accelerometer data points and/or blood pressure data points and location such as GPS data points, agenda item data points. These parameters may be provided by the implantable sensor or any one or more additional implantable sensors and/or other device which is capable of providing data points of one or more of said parameters and/or manual user input. The algorithm executable on the processing means may then be provided for determining trends in the data points of the one or more additional parameters (nutritional intake, activity, location) and generating a personal health profile. In a preferred embodiment the algorithm executable on the processing means may then be provided for determining trends in the data points of the one or more additional parameters (nutritional intake, activity), detecting user dependent correlations between said trends and trends in heart rate data points, trends in creatinine concentration data points and optionally glucose concentration data points, and evaluating said user dependent correlations upon generating a personal health profile. Thus, a further improved personal health profile may be achieved.

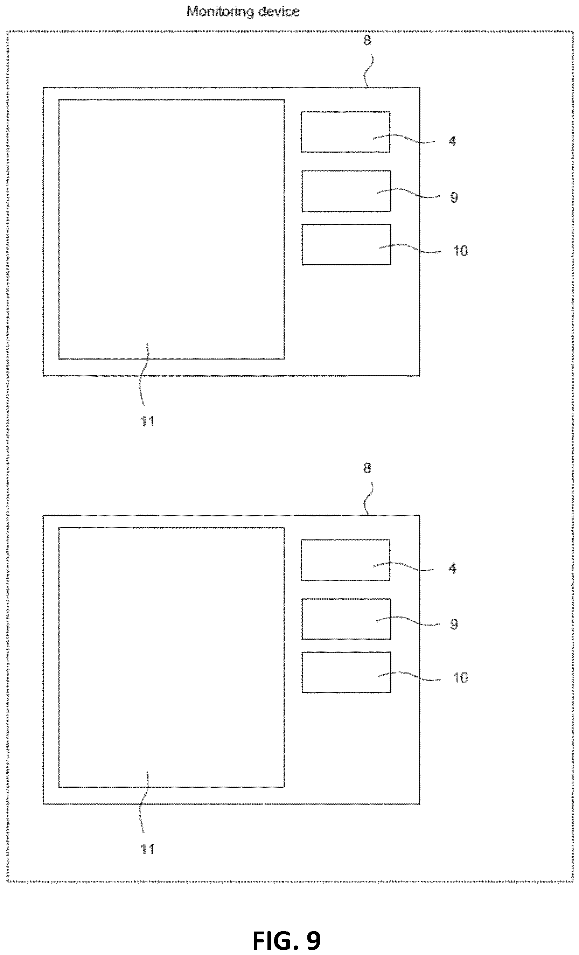

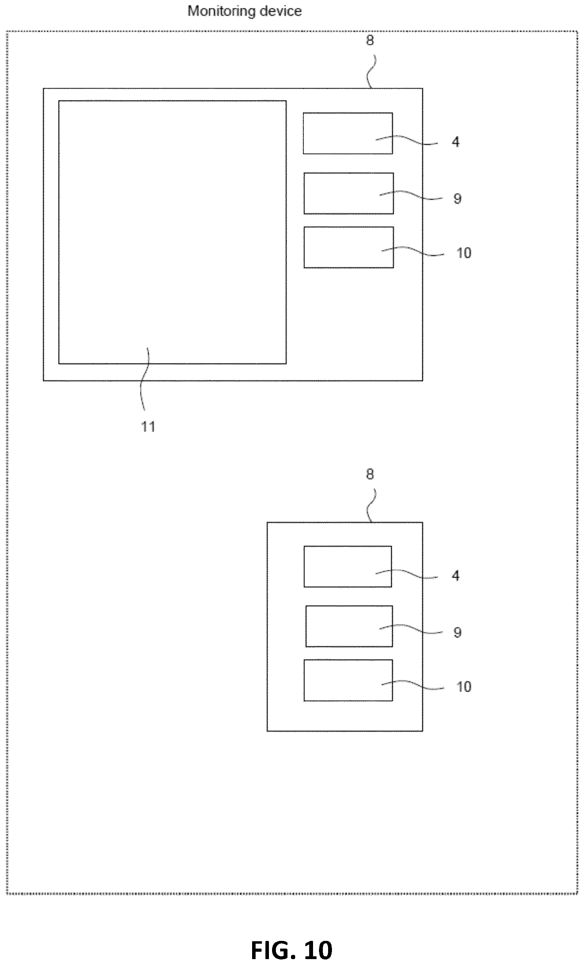

In another embodiment of the invention, different features of the monitoring device may be present on different devices. The monitoring device may thus be an ensemble of two or more devices, each device comprising a transceiver for receiving and transmitting data. Figures 9 and 10 show an embodiment of the invention wherein the monitoring device is an ensemble of devices. In a preferred embodiment the monitoring device comprises one or more of the following devices: a device equipped with a transceiver and a processor, a smartphone and a cloud server. In a more preferred embodiment the device equipped with a transceiver and a processor is capable of transmitting and receiving at least two different communication protocols. In embodiments, the at least two different communication protocols may be wired and/or wireless signals. In embodiments, the wireless signals may comprise signals according to different wireless bands and/or protocols such as IEEE802.11, Bluetooth, cellular etc.

In another embodiment of the invention, the monitoring device may be an ensemble of two or more devices, each device comprising a transceiver for receiving and transmitting data, wherein two or more devices are each equipped with a processing means and an algorithm executable on the processing means. In a preferred embodiment of the invention, the algorithm of each device is provided performing one or more steps necessary for generating a personal health profile according to the invention.

In embodiments the monitoring device may be provided for generating and/or communicating to the user personalized behavioural, lifestyle and therapeutic suggestions and actions, based on the monitoring of biological parameters. In embodiments, the monitoring device may be provided for generating instructions for a controller of an insulin pump or may form part of an insulin pumping device.

In embodiments the monitoring device may be an ensemble of two or more devices wherein each device comprising a transceiver for receiving and transmitting data, wherein two or more devices are each equipped with a processing means and an algorithm executable on the processing means wherein the algorithm of each device is provided for performing one or more steps necessary for generating and/or communicating to the user personalized behavioural, lifestyle and therapeutic suggestions and actions, based on the monitoring of biological parameters. In a preferred embodiment of the invention the monitoring device is an ensemble of devices which includes an insulin pumping device and/or controller of an insulin pump. In a more preferred embodiment of the invention the monitoring device is an ensemble of devices which includes an insulin pumping device and/or controller of an insulin pump and a smartphone.

In a preferred embodiment of the invention, the monitoring device is an ensemble of devices which includes a remote server. In a more preferred embodiment of the invention, the monitoring device is an ensemble of devices which includes a remote server wherein the remote server is equipped with algorithm which is executable on said remote server and provided for performing one or more steps necessary for generating and/or communicating to the user a personal health profile and/or personalized behavioural, lifestyle and therapeutic suggestions and actions, based on the monitoring of biological parameters. In another preferred embodiment of the invention, the monitoring device is an ensemble of devices which does not include a remote server.

A preferred embodiment of the present invention, provides a multiple user health monitoring system comprising a plurality of personal health monitoring systems and further comprising a remote server system which is provided for collecting the personal health profiles generated by the plurality of personal health monitoring systems. In another embodiment, the remote server system is equipped with a further algorithm which is executable on said remote server system and provided for generating reports based on said collected personal health profiles. The reports may include personal recommendations optionally sent back to the monitoring device and displayed to the user, anonymous statistical data from a group of users or instructions for other devices such as an insulin pump. The reports may also include instructions for the monitoring device to be relayed to the implantable sensor.

In embodiments the monitoring device may be provided for generating and/or communicating to the user personalized behavioural, lifestyle and therapeutic advice and/or interventions, based on the monitoring of biological parameters. In a preferred embodiment of the present invention the behavioural, lifestyle and therapeutic suggestions and actions may be one or more of the following: nutritional advice, emergency care advice, therapeutic advice and/or interventions, insulin pump controller instructions.

## Claims

1. A monitoring system (100) for monitoring a kidney-related condition of a living creature, the monitoring system (100) comprising
- an implantable sensor (110) for repetitively measuring creatinine information in bodily tissue or bodily fluids of the living creature and for storing said creatinine information,
- a processor (120) programmed for determining, from the stored information, a kidney-related parameter for the living creature.

2. The monitoring system (100) according to claim 1, wherein the kidney is one of an artificial kidney, a transplanted kidney or an own kidney of the living creature.

3. The monitoring system (100) according to any of the previous claims, wherein the implantable sensor (110) is configured for substantially simultaneously measuring glucose related information in said bodily tissue or bodily fluids and for storing said glucose related information, and
wherein the processor (120) is programmed for determining a kidney-related parameter from the combination of the stored creatinine information and glucose related information.

4. The monitoring system (100) according to claim 3, wherein the implantable sensor (110) is configured for obtaining spectrophotometric sensing data and for deriving the creatinine information and the glucose related information from the same spectrophotometric sensing data.

5. The monitoring system (100) according to any of the previous claims, wherein the monitoring system (100) furthermore comprises an output port for providing an output to a user, a medical practitioner or a medical team and wherein the monitoring system (100) is programmed for providing personalised recommendations regarding the kidney-related parameter to the user through the output port.

6. The monitoring system (100) according to any of the previous claims, wherein the creatinine information is a creatinine concentration and wherein the processor (120) is configured for indicating whether the obtained concentration information is within a predetermined range, for providing the user with a warning for consulting a medical practitioner or for providing a warning to a medical practitioner of the user.

7. The monitoring system (100) according to any of the previous claims, wherein the processor (120) is configured for comparing the creatinine information with a predetermined range, wherein the predetermined range is based on previously measured creatinine information from the living creature.

8. The monitoring system (100) according to any of the previous claims, the processor (120) being adapted for determining an estimated glomerular filtration rate (eGFR).

9. The monitoring system (100) according to claim 8, the living creature being a human being and the system (100) comprising an input port configured for receiving information regarding at least one of an age, length, weight, gender and/or ethnical characteristic of the human being and the processor being configured for taking into account one, more or all of said at least one of age, length, weight, gender and/or ethnical characteristic of the human being for determining the estimated glomerular filtration rate.

10. The monitoring system (100) according to any of the previous claims, wherein the monitoring system (100) is adapted for indicating a degree or stage of diabetes nephropaty or Lupus Nephritis and/or for indicating that a consult with a medical practitioner is advised.

11. The monitoring system (100) according to claim 10, wherein the monitoring system (100) is adapted for detection of a worsening renal function and/or for prognosis of the renal function of the patient.

12. The monitoring system (100) according to any of the previous claims, wherein the implantable sensor (110) is furthermore configured for detecting one or more of lipids, urea or albumin and
wherein the processor (110) is programmed for using the creatinine information in combination with the one or more of lipids, urea or albumin for monitoring chronic kidney disease progression.

13. The monitoring system (100) according to any of the previous claims, wherein the monitoring system (100) is adapted for indicating, after a kidney transplant in a patient, a degree of acceptance of a transplanted kidney by the body of the patient and/or monitoring a graft stability.

14. The monitoring system (100) according to any of the previous claims , wherein the system (100) is configured for time-dependent monitoring during a dialysis session and
wherein the processor (120) is configured for indicating a blood condition as function of the dialysis that is performed in the dialysis session and/or wherein the processor is configured for outputting an indication that, once a blood condition is determined to be appropriate, the dialysis session may be stopped..

15. A method of determining a kidney-related parameter of a living creature, the method comprising repetitively measuring creatinine information in bodily tissue or bodily fluids of the living creature and storing said creatinine information, and determining, from the stored information, a kidney-related parameter for the living creature.
